(19) Europäisches Patentamt
European Patent Office
Office européen des brevets

(11) EP 1 940 373 B1

(12) **EUROPEAN PATENT SPECIFICATION**

(45) Date of publication and mention of the grant of the patent:
**06.05.2015 Bulletin 2015/19**

(21) Application number: **06850456.2**

(22) Date of filing: **13.10.2006**

(51) Int Cl.:
***A61K 31/045*** *(2006.01)* ***A61K 31/047*** *(2006.01)*
***A61K 31/075*** *(2006.01)* ***A61P 25/28*** *(2006.01)*

(86) International application number:
**PCT/IB2006/004181**

(87) International publication number:
**WO 2007/119108 (25.10.2007 Gazette 2007/43)**

(54) **1-FLUORO-1-DEOXY-SCYLLO-INOSITOL FOR THE TREATMENT OF ALZHEIMER'S DISEASE**

1-FLUORO-1-DEOXY-SCYLLO-INOSITOL ZUR BEHANDLUNG VON ALZHEIMER'SCHE KRANKHEIT

1-FLUORO-1-DEOXY-SCYLLO-INOSITOL DESTINE AU TRAITEMENT DE LA MALADIE D'ALZHEIMER

(84) Designated Contracting States:
**AT BE BG CH CY CZ DE DK EE ES FI FR GB GR HU IE IS IT LI LT LU LV MC NL PL PT RO SE SI SK TR**

(30) Priority: **13.10.2005 US 725634 P**

(43) Date of publication of application:
**09.07.2008 Bulletin 2008/28**

(73) Proprietor: **Waratah Pharmaceuticals, Inc.**
**Toronto, ON M5G 1L7 (CA)**

(72) Inventors:
- **CRUZ, Antonio**
  **Toronto, Ontario M5P 2T7 (CA)**
- **KURDYDYK, Linda**
  **Toronto, Ontario M4R 1H3 (CA)**

(74) Representative: **Holliday, Louise Caroline et al**
**D Young & Co LLP**
**120 Holborn**
**London EC1N 2DY (GB)**

(56) References cited:
**WO-A-2007/041855** **WO-A-2007/134449**
**WO-A1-2004/075882** **WO-A1-2006/053428**
**CA-A1- 2 214 635** **CA-A1- 2 486 663**

- **SUN Y ET AL: "Synthesis of scyllo-inositol derivatives and their effects on amyloid beta peptide aggregation", BIOORGANIC & MEDICINAL CHEMISTRY, PERGAMON, GB, vol. 16, no. 15, 1 August 2008 (2008-08-01), pages 7177-7184, XP023610694, ISSN: 0968-0896, DOI: 10.1016/J.BMC.2008.06.045 [retrieved on 2008-06-26]**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 940 373 B1

## Description

The invention relates to compounds and compositions for treating Alzheimer's disease, which characterized by abnormal protein folding or aggregation or amyloid formation, desposition, accumulation or persistence.

## BACKGROUND OF INVENTION

Scyllo-inositol is one of the nine known stereoisomers of hexahydroxycyclohexane (Bouveault L. Bull. La Societe Chimique Paris 1894: 11: 44-147). The compound is present in human brain in quantities estimated to from 5 to 12 % that of myo-inositol (5 mM) (Michaelis T et al. NMR in Biomedicien 1993: 6: 105-109). WO 2004/075882 published September 10, 2004 discloses the use of scyllo-inosital in the prevention and treatment of disorders in protein folding or aggregation, or amyloid formation, deposition, accumulation, or persistence.

## SUMMARY OF INVENTION

Broadly stated, the invention provides a pharmaceutical composition for use in the treatment or prevention of Alzheimer's disease comprising a therapeutically effective amount of a compound of the formula II

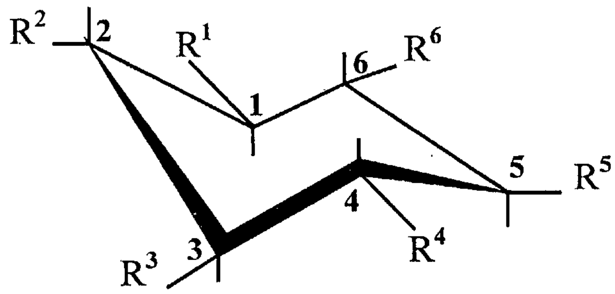

II

wherein five of $R^1$, $R^2$, $R^3$, $R^4$, $R^5$, and $R^6$ are hydroxyl and one of $R^1$, $R^2$, $R^3$, $R^4$, $R^5$, and $R^6$ is fluoro.

In another aspect, the invention relates to use of a pharmaceutical composition or a therapeutically effective amount of a compound of the formula II as defined above in the manufacture of a medicament for the treatment or prevention of Alzheimer's disease in a subject.

These and other aspects, features, and advantages of the present invention should be apparent to those skilled in the art from the following detailed description.

## DETAILED DESCRIPTION OF EMBODIMENTS

For convenience, certain terms employed in the specification, examples, and appended claims are collected here.

The recitation of numerical ranges by endpoints herein includes all numbers and fractions subsumed within that range (e.g. 1 to 5 includes 1, 1.5, 2, 2.75, 3, 3.90, 4, and 5). It is also to be understood that all numbers and fractions thereof are presumed to be modified by the term "about." The term "about" means plus or minus 0.1 to 50%, 5-50%, or 10-40%, preferably 10-20%, more preferably 10% or 15%, of the number to which reference is being made. Further, it is to be understood that "a," "an," and "the" include plural referents unless the content clearly dictates otherwise. Thus, for example, reference to a composition comprising "a compound" includes a mixture of two or more compounds.

The terms "administering" and "administration" refer to the process by which a therapeutically effective amount of a compound or composition contemplated herein is delivered to a subject for prevention and/or treatment purposes. Compositions are administered in accordance with good medical practices taking into account the subject's clinical condition, the site and method of administration, dosage, patient age, sex, body weight, and other factors known to physicians.

The term "treating" refers to reversing, alleviating, or inhibiting the progress of a disease, or one or more symptoms of such disease, to which such term applies. Treating includes the management and care of a subject at diagnosis or later. A treatment may be either performed in an acute or chronic way. Depending on the condition of the subject, the term also refers to preventing a disease, and includes preventing the onset of a disease, or preventing the symptoms associated with a disease. The term also refers to reducing the severity of a disease or symptoms associated

with such disease prior to affliction with the disease. Such prevention or reduction of the severity of a disease prior to affliction refers to administration of a compound or composition of the present invention to a subject that is not at the time of administration afflicted with the disease. "Preventing" also refers to preventing the recurrence of a disease or of one or more symptoms associated with such disease. An objective of treatment is to combat the disease and includes administration of the active compounds to prevent or delay the onset of the symptoms or complications, or alleviating the symptoms or complications, or eliminating or partially eliminating the condition and/or disease. The terms "treatment" and "therapeutically," refer to the act of treating, as "treating" is defined above.

The terms "subject", "individual", or "patient" are used interchangeably herein and refer to an animal including a warm-blooded animal such as a mammal. Mammal includes without limitation any members of the Mammalia. In general, the terms refer to a human. The terms also include domestic animals bred for food or as pets, including horses, cows, sheep, poultry, fish, pigs, cats, dogs, and zoo animals, goats, apes (e.g. gorilla or chimpanzee), and rodents such as rats and mice. Typical subjects for treatment include persons afflicted with or suspected of having or being pre-disposed to a disease disclosed herein, or persons susceptible to, suffering from or that have suffered a disease described herein. A subject may or may not have a genetic predisposition for a disease disclosed herein such as Alzheimer's disease. In particular aspects, a subject shows signs of cognitive deficits and amyloid plaque neuropathology. In embodiments of the invention the subjects are suspectible to, or suffer from Alzheimer's disease.

As utilized herein, the term "healthy subject" means a subject, in particular a mammal, having no diagnosed disease, disorder, infirmity, or ailment known to impair or otherwise diminish memory.

A "beneficial effect" refers to an effect of a compound of the invention or composition thereof in certain aspects of the invention, including favorable pharmacological and/or therapeutic effects, and improved biological activity. In aspects of the invention, the beneficial effects include without limitation prevention, reduction or inhibition of A fibril assembly or aggregation, Aβ toxicity, Aβ42 levels, abnormal protein folding, aggregation, amyloid formation, deposition, accumulation or persistence, and/or amyloid lipid interactions, and/or acceleration of disassembly of preformed fibrils. In particular embodiments of the invention, the beneficial effects include but are not limited to the following: disruption of aggregated Aβ; increased inhibition of long term potentiation induced by Aβ oligomers; maintenance of synaptic function; inhibition of Aβ-induced progressive cognitive decline and cerebral amyloid plaque pathology; improved cognition; increased lifespan; reduced cerebral accumulation of Aβ; reduced deposition of cerebral amyloid plaques; reduced soluble Aβ oligomers (e.g. Aβ42) in the brain; reduced glial activity; reduced inflammation; and/or cognitive decline. In some aspects, a beneficial effect is a favourable characteristic of a composition/formulation of the invention includes enhanced stability, a longer half life, and/or enhanced uptake and transport across the blood brain barrier.

The beneficial effect may be a statistically significant effect in terms of statistical analysis of an effect of a compound of the invention versus the effects without the compound or an inositol compound that is not within the scope of the invention (e.g. myo-inositol or unmodified scyllo-inositol). "Statistically significant" or "significantly different" effects or levels may represent levels that are higher or lower than a standard. In embodiments of the invention, the difference may be 1.5, 2, 3, 4, 5, or 6 times higher or lower compared with the effect obtained without a compound of the invention.

The term "pharmaceutically acceptable carrier, excipient, or vehicle" refers to a medium which does not interfere with the effectiveness or activity of an active ingredient and which is not toxic to the hosts to which it is administered. A carrier, excipient, or vehicle includes diluents, binders, adhesives, lubricants, disintegrates, bulking agents, wetting or emulsifying agents, pH buffering agents, and miscellaneous materials such as absorbants that may be needed in order to prepare a particular composition. Examples of carriers etc. include saline, buffered saline, dextrose, water, glycerol, ethanol, and combinations thereof. The use of such media and agents for an active substance is well known in the art.

"Pharmaceutically acceptable salt(s)," means a salt that is pharmaceutically acceptable and has the desired pharmacological properties. By pharmaceutically acceptable salts is meant those salts which are suitable for use in contact with the tissues of a subject or patient without undue toxicity, irritation and allergic response, and are commensurate with a reasonable benefit/risk ratio. Pharmaceutically acceptable salts are described for example, in S. M. Berge, et al., J. Pharmaceutical Sciences, 1977, 66:1. Suitable salts include salts that may be formed where acidic protons in the compounds are capable of reacting with inorganic or organic bases. Suitable inorganic salts include those formed with alkali metals, e.g. sodium and potassium, magnesium, calcium, and aluminum. Suitable organic salts include those formed with organic bases such as the amine bases, e.g. ethanolamine, diethanolamine, triethanolamine, tromethamine and N-methylglucamine. Suitable salts also include acid addition salts formed with inorganic acids (e.g. hydrochloric and hydrobromic acids) and organic acids (e.g. acetic acid, citric acid, maleic acid, and the alkane- and arene-sulfonic acids such as methanesulfonic acid and benezenesulfonic acid). When there are two acidic groups present, a pharmaceutically acceptable salt may be a mono-acid-mono-salt or a disalt; and similarly where there are more than two acidic groups present, some or all of such groups can be salified.

"Therapeutically effective amount" relates to the amount or dose of an active compound or composition of the invention that will lead to one or more desired effects, in particular, one or more beneficial effects. A therapeutically effective amount of a substance can vary according to factors such as the disease state, age, sex, and weight of the subject, and the ability of the substance to elicit a desired response in the subject. A dosage regimen may be adjusted

to provide the optimum therapeutic response (e.g. sustained beneficial effects). For example, several divided doses may be administered daily or the dose may be proportionally reduced as indicated by the exigencies of the therapeutic situation.

As used "nutraceutically acceptable derivative" refers to a derivative or substitute for the stated chemical species that operates in a similar manner to produce the intended effect, and is structurally similar and physiologically compatible. Examples of substitutes include salts, esters, hydrates, or complexes of the stated chemical. The substitute could also be a precursor or prodrug to the stated chemical, which subsequently undergoes a reaction *in vivo* to yield the stated chemical or a substitute thereof.

The term "pure" in general means better than 90%, 92%, 93%, 94%, 95%, 96%, 97%, 98% or 99% pure, and "substantially pure" means a compound synthesized such that the compound, as made as available for consideration into a composition or therapeutic dosage of the invention, has only those impurities that can not readily nor reasonably be removed by conventional purification processes.

The term "hydroxyl" or "hydroxy" refers to a single -OH group.

In connection with the present invention, the disease is Alzheimer's disease including familial and non-familial types.

### Compound

In the pharmaceutical composition for use according to the invention, five of $R^1$, $R^2$, $R^3$, $R^4$, $R^5$, or $R^6$ of the compound of formula II are hydroxyl and the other of $R^1$, $R^2$, $R^3$, $R^4$, $R^5$, or $R^6$ is fluoro.

A compound of the invention may additionally comprise a carrier, including with out limitation one or more of a polymer, carbohydrate, peptide or derivative thereof. A carrier may be substituted with substituents described herein including one or more alkyl, amino, nitro, halogen, thiol, thioalkyl, sulfate, sulfonyl, sulfenyl, sulfinyl, sulfoxide, hydroxyl groups. A carrier can be directly or indirectly covalently attached to a compound of the invention. In aspects of the invention the carrier is an amino acid including alanine, glycine, praline, methionine, serine, threonine, or asparagine. In other aspects the carrier is a peptide including alanyl-alanyl, prolyl-methionyl, or glycyl-glycyl.

A carrier also includes a molecule that targets a compound of the invention to a particular tissue or organ. In particular, a carrier may facilitate or enhance transport of a compound of the invention to the brain by either active or passive transport.

### Process

The compounds of the formula II for use according to this invention may be prepared using reactions and methods generally known to the person of ordinary skill in the art, having regard to that knowledge and the disclosure of this application including the Examples. The reactions are performed in a solvent appropriate to the reagents and materials used and suitable for the reactions being effected. It will be understood by those skilled in the art of organic synthesis that the functionality present on the compounds should be consistent with the proposed reaction steps. This will sometimes require modification of the order of the synthetic steps or selection of one particular process scheme over another in order to obtain a desired compound of the invention. It will also be recognized that another major consideration in the development of a synthetic route is the selection of the protecting group used for protection of the reactive functional groups present in the compounds described in this invention. An authoritative account describing the many alternatives to the skilled artisan is Greene and Wuts (Protective Groups In Organic Synthesis, Wiley and Sons, 1991).

The starting materials and reagents used in preparing compounds for use in the invention are either available from commercial suppliers such as the Aldrich Chemical Company (Milwaukee, Wis.), Bachem (Torrance, Calif.), Sigma (St. Louis, Mo.), or Lancaster Synthesis Inc. (Windham, N.H.) or are prepared by methods well known to a person of ordinary skill in the art, following procedures described in such references as Fieser and Fieser's Reagents for Organic Synthesis, vols. 1-17, John Wiley and Sons, New York, N.Y., 1991; Rodd's Chemistry of Carbon Compounds, vols. 1-5 and supps., Elsevier Science Publishers, 1989; Organic Reactions, vols. 1-40, John Wiley and Sons, New York, N.Y., 1991; March J.: Advanced Organic Chemistry, 4th ed., John Wiley and Sons, New York, N.Y.; and Larock: Comprehensive Organic Transformations, VCH Publishers, New York, 1989. Publications disclosing particular processes for preparing scyllo-inositol include Husson, C., et al, Carbohyrate Research 307 (1998) 163-165) and Sarmah, M.P. and Shashidar, M.S., Carbohydrate Research 338 (2003) 999-1001.

The starting materials, intermediates, and compounds for use in this invention may be isolated and purified using conventional techniques, such as precipitation, filtration, distillation, crystallization, chromatography, and the like. The compounds may be characterized using conventional methods, including physical constants and spectroscopic methods, in particular HPLC.

The compounds of the formula II which are basic in nature can form a wide variety of different salts with various

inorganic and organic acids. In practice is it desirable to first isolate a compound of the formula I from the reaction mixture as a pharmaceutically unacceptable salt and then convert the latter to the free base compound by treatment with an alkaline reagent and subsequently convert the free base to a pharmaceutically acceptable acid addition salt. The acid addition salts of the base compounds of this invention are readily prepared by treating the base compound with a substantially equivalent amount of the chosen mineral or organic acid in an aqueous solvent medium or in a suitable organic solvent such as methanol or ethanol. Upon careful evaporation of the solvent, the desired solid salt is obtained.

Compounds of the formula II which are acidic in nature are capable of forming base salts with various pharmacologically acceptable cations. These salts may be prepared by conventional techniques by treating the corresponding acidic compounds with an aqueous solution containing the desired pharmacologically acceptable cations and then evaporating the resulting solution to dryness, preferably under reduced pressure. Alternatively, they may be prepared by mixing lower alkanolic solutions of the acidic compounds and the desired alkali metal alkoxide together and then evaporating the resulting solution to dryness in the same manner as before. In either case, stoichiometric quantities of reagents are typically employed to ensure completeness of reaction and maximum product yields.

## Compositions and Kits

A compound of the formula II for use in the invention may be formulated into a pharmaceutical composition or dietary supplement for administration to a subject. Pharmaceutical compositions for use in the present invention or fractions thereof comprise suitable pharmaceutically acceptable carriers, excipients, and vehicles selected based on the intended form of administration, and consistent with conventional pharmaceutical practices.

Suitable pharmaceutical carriers, excipients, and vehicles are described in the standard text, Remington's Pharmaceutical Sciences, Mack Publishing Company. By way of example for oral administration in the form of a capsule or tablet, the active components can be combined with an oral, non-toxic pharmaceutically acceptable inert carrier such as lactose, starch, sucrose, methyl cellulose, magnesium stearate, glucose, calcium sulfate, dicalcium phosphate, mannitol and sorbital. For oral administration in a liquid form, the drug components may be combined with any oral, non-toxic, pharmaceutically acceptable inert carrier such as ethanol, glycerol and water. Suitable binders (e.g. gelatin, starch, corn sweeteners, natural sugars including glucose; natural and synthetic gums, and waxes), lubricants (e.g. sodium oleate, sodium stearate, magnesium stearate, sodium benzoate, sodium acetate, and sodium chloride), disintegrating agents (e.g. starch, methyl cellulose, agar, bentonite, and xanthan gum), flavoring agents, and coloring agents may also be combined in the compositions or components thereof. Compositions as described herein can further comprise wetting or emulsifying agents, or pH buffering agents.

A composition of the invention can be a liquid solution, suspension, emulsion, tablet, pill, capsule, sustained release formulation, or powder. The compositions can be formulated as a suppository, with traditional binders and carriers such as triglycerides. Oral formulations can include standard carriers such as pharmaceutical grades of mannitol, lactose, starch, magnesium stearate, sodium saccharine, cellulose and magnesium carbonate. Various delivery systems are known and can be used to administer a composition of the invention, e.g. encapsulation in liposomes, microparticles and microcapsules.

Formulations for parenteral administration may include aqueous solutions, syrups, aqueous or oil suspensions and emulsions with edible oil such as cottonseed oil, coconut oil or peanut oil. Dispersing or suspending agents that can be used for aqueous suspensions include synthetic or natural gums, such as tragacanth, alginate, acacia, dextran, sodium carboxymethylcellulose, gelatin, methylcellulose, and polyvinylpyrrolidone.

Compositions for parenteral administration may include sterile aqueous or non-aqueous solvents, such as water, isotonic saline, isotonic glucose solution, buffer solution, or other solvents conveniently used for parenteral administration of therapeutically active agents. A composition intended for parenteral administration may also include conventional additives such as stabilizers, buffers, or preservatives, e.g. antioxidants such as methylhydroxybenzoate or similar additives.

Compositions of the invention can be formulated as pharmaceutically acceptable salts as described herein.

A composition of the invention may be sterilized by, for example, filtration through a bacteria retaining filter, addition of sterilizing agents to the composition, irradiation of the composition, or heating the composition. Alternatively, the compounds or compositions of the present invention may be provided as sterile solid preparations e.g. lyophilized powder, which are readily dissolved in sterile solvent immediately prior to use.

After pharmaceutical compositions have been prepared, they can be placed in an appropriate container and labeled for treatment of an indicated condition. For administration of a composition, such labeling would include amount, frequency, and method of administration.

A compound of the formula II may be in a form suitable for administration as a dietary supplement. A supplement may optionally include inactive ingredients such as diluents or fillers, viscosity-modifying agents, preservatives, flavorings, colorants, or other additives conventional in the art. By way of example only, conventional ingredients such as beeswax, lecithin, gelatin, glycerin, caramel, and carmine may be included.

A dietary supplement composition for use in the invention may optionally comprise a second active ingredient. In an embodiment, the second active ingredient is pinitol or an active derivative or metabolite thereof. Pinitol can be produced from plant sources, including alfalfa, Bougainvillea leaves, chick peas, pine trees and soy beans. Pinitol is also commercially available, for example Inzitol™ (Humanetics Corporation, Min). Examples of derivatives and metabolites of pinitol include pinitol glycosides, pinitol phospholipids, esterified pinitol, lipid-bound pinitol, pinitol phosphates, pinitol phytates, and hydrolyzed pinitol such as d-chiro-inositol.

A dietary supplement may be provided as a liquid dietary supplement e.g., a dispensable liquid) or alternatively the compositions may be formulated as granules, capsules or suppositories. The liquid supplement may include a number of suitable carriers and additives including water, glycols, oils, alcohols, flavoring agents, preservatives and coloring agents. In capsule, granule or suppository form, the compositions of the present invention are formulated in admixture with a pharmaceutically acceptable carrier.

A supplement may be presented in the form of a softgel which is prepared using conventional methods. A softgel typically includes a layer of gelatin encapsulating a small quantity of the supplement. A supplement may also be in the form of a liquid-filled and sealed gelatin capsule, which may be made using conventional methods.

To prepare a dietary supplement composition in capsule, granule or suppository form, one or more compositions for use in the present invention may be intimately admixed with a pharmaceutically acceptable carrier according to conventional formulation techniques. For solid oral preparations such as capsules and granules, suitable carriers and additives such as starches, sugars, diluents, granulating agents, lubricants, binders and disintegrating agents may be included.

For use in the invention, a pharmaceutical pack or kit may be provided comprising one or more containers filled with one or more of the ingredients of a pharmaceutical composition for use in the invention to provide a beneficial effect, in particular a sustained beneficial effect. Associated with such container(s) can be various written materials such as instructions for use, or a notice in the form prescribed by a governmental agency regulating the labeling, manufacture, use or sale of pharmaceuticals or biological products, which notice reflects approval by the agency of manufacture, use, or sale for human administration.

A kit may be provided whcih kit comprises a compound or a pharmaceutical composition ofr use in the invention. The kit can be a package which houses a container which contains a composition for use in the invention and also houses instructions for administering the composition to a subject.

## Applications

The present invention in embodiments may provide a composition comprising a compound that provides beneficial effects including greater solubility, stability, efficacy, potency, and/or utility, in particular greater solubility and stability.

In the invention a compound of the formula II is for use in the treatment of Alzheimer's disease. Thus, Alzheimer's disease may be treated by administering a therapeutically effective amount of a compound of the formula II. Such treatment may be effective for retarding the degenerative effects of Alzheimer's disease, including specifically, but not exclusively, deterioration of the central nervous system, loss of mental facilities, loss of short term memory, and disorientation.

As the disease is Alzheimer's disease, beneficial effects of a composition for use in the invention can manifest as one, two, three, four, five, six, seven, eight, nine, or all of the following, in particular five or more, more particularly 8 or more of the following:

a) An increase or restoration of long term potentiation relative to the level in the absence of a compound disclosed herein after administration to a subject with symptoms of Alzheimer's disease. In aspects of the invention a compound disclosed herein induces at least about a 0.05%, 0.1%, 0.5%, 1%, 2%, 5%, 10%, 15%, 20%, 30%, 33%, 35%, 40%, 45%, 50%, 60%, 70%, 80%, 90%, 95%, or 99% increase in long term potentiation in a subject.

b) An increase or maintenance of synaptic function relative to the level of synaptic function in the absence of a compound disclosed herein after administration to a subject with symptoms of Alzheimer's disease. In aspects of the invention a compound disclosed herein induces at least about a 0.05%, 0.1%, 0.5%, 1%, 2%, 5%, 10%, 15%, 20%, 30%, 33%, 35%, 40%, 45%, 50%, 60%, 70%, 80%, 90%, 95%, 99%, 100%, 125%, 150%, 175% or 200% increase in synaptic function in a subject.

c) An increase in synaptophysin. In aspects of the invention there is at least about a 2%, 5%, 10%, 15%, 20%, 30%, 40%, 50%, 60%, 70%, 80%, 90%, 95%, 99%, 100%, 125%, 150%, 175% or 200% increase in synaptophysin.

d) An increase in synaptophysin reactive boutons and cell bodies. In aspects of the invention there is at least about a 2%, 5%, 10%, 15%, 20%, 30%, 40%, 50%, 60%, 70%, 80%, 90%, 95%, 99%, 100%, 125%, 150%, 175% or 200%, more particularly about a 100-150% or 140-150% increase in synaptophysin reactive boutons and cell bodies,.

e) A reduction, slowing or prevention of an increase in, or an absence of symptoms of inflammation, in particular

an Aβ-induced inflammatory response, after administration to a subject with symptoms of Alzheimer's disease.

f) A reduction, slowing or prevention of an increase in cerebral accumulation of amyloid β relative to the levels measured in the absence of a compound disclosed herein in subjects with symptoms of Alzheimer's disease. In aspects of the invention, the compound induces at least about a 2%, 5%, 10%, 15%, 20%, 30%, 40%, 50%, 60%, 70%, 80%, or 90% decrease in cerebral accumulation of amyloid P.

g) A reduction, slowing or prevention of an increase in deposition of cerebral amyloid plaques, relative to the levels measured in the absence of a compound disclosed herein in subjects with symptoms of Alzheimer's disease. In aspects of the invention, the compound induces at least about a 2%, 5%, 10%, 15%, 20%, 30%, 40%, 50%, 60%, 70%, 80%, or 90% decrease in deposition of cerebral amyloid plaques.

h) A reduction, slowing or prevention of an increase in plaque number. In aspects of the invention, a compound disclosed herein induces at least about a 2%, 5%, 10%, 15%, 20%, 30%, 40%, 50%, 60%, 70%, 80%, or 90% reduction in plaque number. In particular aspects the compound induces a 5-15% or 10-15% reduction in plaque number.

i) A reduction, slowing or prevention of an increase in plaque size. In aspects of the invention, a compound disclosed herein induces at least about a 2%, 5%, 10%, 15%, 20%, 30%, 40%, 50%, 60%, 70%, 80%, or 90% reduction in plaque size. In particular aspects the compound induces a 5-15% or 10-15% reduction in plaque size.

j) A reduction, slowing or prevention of an increase in percent area of the brain covered in plaques. In aspects of the invention, a compound disclosed herein induces at least about a 2%, 5%, 10%, 15%, 20%, 30%, 40%, 50%, 60%, 70%, 80%, or 90% reduction in percent area of the brain covered in plaques. In particular aspects the compound induces a 5-15% or 10-15% reduction in percent area of the brain covered in plaques.

k) A reduction, slowing or prevention of an increase in soluble Aβ oligomers in the brain, relative to the levels measured in the absence of a compound disclosed herein in subjects with symptoms of Alzheimer's disease. In aspects of the invention, the combination induces at least about a 2%, 5%, 10%, 15%, 20%, 30%, 40%, 50%, 60%, 70%, 80%, or 90% decrease in soluble Aβ oligomers.

l) A reduction, slowing or prevention of an increase in brain levels of Aβ40. In aspects of the invention, a compound disclosed herein induces at least about a 2%, 5%, 10%, 15%, 20%, 30%, 40%, 50%, 60%, 70%, 80%, or 90% reduction in Aβ40. In particular aspects the compound induces a 10-50%, 20-45%, or 25-35% reduction in brain levels of Aβ40.

m) A reduction, slowing or prevention of an increase in Aβ42 levels in a body fluid such as CSF or blood. In aspects of the invention, a compound disclosed herein induces at least about a 2%, 5%, 10%, 15%, 20%, 30%, 40%, 50%, 60%, 70%, 80%, or 90% reduction in Aβ42. In particular aspects the compound induces a 10-50%, 15-40%, or 20-25% reduction in brain levels of Aβ42.

n) A reduction, slowing or prevention of an increase in brain levels of Aβ42. In aspects of the invention, a compound disclosed herein induces at least about a 2%, 5%, 10%, 15%, 20%, 30%, 40%, 50%, 60%, 70%, 80%, or 90% reduction in Aβ42. In particular aspects the compound induces a 10-50%, 15-40%, or 20-25% reduction in brain levels of Aβ42.

o) A reduction, slowing or prevention of an increase in glial activity in the brain, relative to the levels measured in the absence of a compound disclosed herein in subjects with symptoms of Alzheimer's disease. Preferably, the compound induces at least about a 2%, 5%, 10%, 15%, 20%, 30%, 40%, 50%, 60%, 70%, 80%, or 90% decrease in glial activity

p) Maintenance of synaptic function at about normal for a prolonged period of time, in particular for at least 5 weeks, 6 weeks, 8 weeks, 10 weeks, 12 weeks, 14 weeks, 16 weeks, 20 weeks, 24 weeks, 30 weeks, 40 weeks, 52 weeks, or 78 weeks, more particularly, 2 to 4 weeks, 2 to 5 weeks, 3 to 5 weeks, 2 to 6 weeks, 2 to 8 weeks, 2 to 10 weeks, 2 to 12 weeks, 2 to 16 weeks, 2 to 20 weeks, 2 to 24 weeks, 2 weeks to 12 months, or 2 weeks to 24 months following treatment.

q) A reduction or slowing of the rate of disease progression in a subject with Alzheimer's disease. In particular a reduction or slowing of cognitive decline in a subject with Alzheimer's disease.

r) A reduction, slowing or prevention of an increase in cognitive deficits.

s) A reduction, slowing or prevention of an increase in amyloid angiopathy.

t) A reduction in accelerated mortality.

u) An increase in survival in a subject with symptoms of Alzheimer's disease.

In aspects of the invention beneficial effects of a composition or treatment of the invention can manifest as (a) and (b); (a), (b) and (c); (a), (b), (e), (f) and (g); (a), (b), (e), (f) through (h); (a), (b), (e), (f) through (i); (a), (b), (e), (f) through (j); (a), (b), (e), (f) through (k); (a), (b), (e), (f) through (l); (a), (b), (e), (f) through (m); (a), (b), (e), (f) through (n); (a), (b), (e), (f) through (o); (a), (b), (e), (f) through (p); (a), (b), (e), (f) through (q); (a), (b), (e), (f) through (r); (a), (b), (e), (f) through (s); (a), (b), (e), (f) through (t); (a) through (d); (a) through (e); (a) through (f); (a) through (g); (a) through (h); (a) through (i); (a) through (j); (a) through (k); (a) through (l); (a) through (m); (a) through (n); (a) through (o); (a) through

(p); (a) through (q); (a) through (r); (a) through (s); (a) through (t), or (a) through (u).

Pharmaceutical compositions for use in the invention can be selected that have sustained beneficial effects, preferably statistically significant sustained beneficial effects.

Greater efficacy and potency of a treatment may improve the therapeutic ratio of treatment, reducing untoward side effects and toxicity. The treatment may also improve long-standing Alzheimer's disease even when treatment is begun long after the appearance of symptoms. Prolonged efficacious treatment can be achieved following administration of a compound or composition as defined herein.

The treatment of Alzheimer's disease may comprise contacting Aβ or Aβ aggregates, in particular Aβ40 or Aβ40 aggregates and/or Aβ42 or Aβ42 aggregates, in a subject with a therapeutically effective amount of a compound or a composition as defined herein.

The treatment of Alzheimer's disease may involve providing a composition comprising a compound as defined herein in an amount sufficient to disrupt aggregated Aβ for a prolonged period following administration.

The treatment of Alzheimer's disease in a patient in need thereof which may include administering to the individual a composition that provides a compound as defined herein in a dose sufficient to increase inhibition of long term potentiation induced by Aβ oligomers and/or maintain synaptic function. The treatment of Alzheimer's disease may comprise administering, preferably orally or systemically, an amount of a compound as defined herein to a mammal, to reduce cerebral accumulation of Aβ, deposition of cerebral amyloid plaques, soluble Aβ oligomers in the brain, glial activity, and/or inflammation for a prolonged period following administration.

The treatment of Alzheimer's disease, may comprise administering to a mammal in need thereof a composition comprising a compound as defined herein in an amount sufficient to reduce cognitive decline for a prolonged period following administration, thereby treating the Alzheimer's disease.

The treatment of Alzheimer's disease, may comprise administering to a mammal in need thereof a composition comprising a compound as defined herein in an amount sufficient to disrupt aggregated Aβ for a prolonged period following administration; and determining the amount of aggregated Aβ, thereby treating the Alzheimer's disease. The amount of aggregated Aβ may be measured using an antibody specific for Aβ or a compound as defined herein labeled with a detectable substance.

The compositions as defined herein may be used in combination with one or more additional therapeutic agents including beta-secretase inhibitors, alpha-secretase inhibitors, and epsilon-secretase inhibitors, agents that are used for the treatment of complications resulting from or associated with a disease, or general medications that treat or prevent side effects.

The invention also contemplates the use of a pharmaceutical composition or a therapeutically effective amount of at least one compound as defined herein for preparation of a medicament for providing therapeutic effects, in particular beneficial effects, preferably sustained beneficial effects, in treating Alzheimer's disease.

Therapeutic efficacy and toxicity of compositions and methods of the invention may be determined by standard pharmaceutical procedures in cell cultures or with experimental animals such as by calculating a statistical parameter such as the $ED_{50}$ (the dose that is therapeutically effective in 50% of the population) or $LD_{50}$ (the dose lethal to 50% of the population) statistics. The therapeutic index is the dose ratio of therapeutic to toxic effects and it can be expressed as the $ED_{50}/LD_{50}$ ratio. Pharmaceutical compositions which exhibit large therapeutic indices are preferred. By way of example, one or more of the therapeutic effects, in particular beneficial effects disclosed herein, can be demonstrated in a subject or disease model, for example, a TgCRND8 mouse with symptoms of Alzheimer's disease.

**Administration**

Compounds and compositions for use in the present invention can be administered by any means that produce contact of the active agent(s) with the agent's sites of action in the body of a subject or patient to produce a therapeutic effect, in particular a beneficial effect, in particular a sustained beneficial effect. The active ingredients can be administered simultaneously or sequentially and in any order at different points in time to provide the desired beneficial effects. A compound and composition for use in the invention can be formulated for sustained release, for delivery locally or systemically. It lies within the capability of a skilled physician or veterinarian to select a form and route of administration that optimizes the effects of the compositions and treatments of the present invention to provide therapeutic effects, in particular beneficial effects, more particularly sustained beneficial effects.

The compositions may be administered in oral dosage forms such as tablets, capsules (each of which includes sustained release or timed release formulations), pills, powders, granules, elixirs, tinctures, suspensions, syrups, and emulsions. They may also be administered in intravenous (bolus or infusion), intraperitoneal, subcutaneous, or intramuscular forms, all utilizing dosage forms well known to those of ordinary skill in the pharmaceutical arts. The compositions of the invention may be administered by intranasal route via topical use of suitable intranasal vehicles, or via a transdermal route, for example using conventional transdermal skin patches. A dosage protocol for administration using a transdermal delivery system may be continuous rather than intermittent throughout the dosage regimen. A sustained release formulation can also be used for the therapeutic agents.

The dosage regimen of the invention will vary depending upon known factors such as the pharmacodynamic characteristics of the agents and their mode and route of administration; the species, age, sex, health, medical condition, and weight of the patient, the nature and extent of the symptoms, the kind of concurrent treatment, the frequency of treatment, the route of administration, the renal and hepatic function of the patient, and the desired effect.

An amount of a therapeutic of the invention which will be effective in the treatment of a particular disorder or disease to provide effects, in particular beneficial effects, more particularly sustained beneficial effects, will depend on the nature of the condition or disorder, and can be determined by standard clinical techniques. The precise dose to be employed in the formulation will also depend on the route of administration, and the seriousness of the disease, and should be decided according to the judgement of the practitioner and each patient's circumstances.

Suitable dosage ranges for administration are particularly selected to provide therapeutic effects, in particular beneficial effects, more particularly sustained beneficial effects. A dosage range is generally effective for triggering the desired biological responses. The dosage ranges are generally about 0.1 mg to about 2 kg per kg per day, about 0.5 mg to about 2 g per kg per day, about 1 mg to about 1 g per kg per day, about 1 mg to about 200 mg per kg per day, about 1 mg to about 100 mg per kg per day, about 10 mg to about 100 mg per kg, 30 mg to 70 mg per kg per day, about 1 mg to about 50 mg per kg per day, about 2 to about 50 mg/kg/day, about 2 mg to about 40 mg per kg, or about 3 mg to 30 mg per kg per day. In aspects of the invention, the dosage ranges are generally about .5 mg to about 2 g per kg, about 1 mg to about 1 g per kg, about 1 mg to about 200 mg per kg, about 1 mg to about 100 mg per kg, about 1 mg to about 50 mg per kg, about 10 mg to about 100 mg per kg, or about 30 mg to 70 mg per kg of the weight of a subject.

In some aspects of the invention, the dosage ranges of a compound disclosed herein administered once twice, three times or more daily, especially once or twice daily, are about 1 to 100 mg/kg, 1 to 90 mg/kg, 1 to 80 mg/kg, 1 to 75 mg/kg, 1 to 70 mg/kg, 1 to 60 mg/kg, 1 to 50 mg/kg, 1 to 40 mg/kg, 1 to 35 mg/kg, 2 to 35 mg/kg, 2.5 to 30 mg/kg, 3 to 30 mg/kg, 3 to 20 mg/kg, or 3 to 15 mg/kg. In embodiments of the invention, the required dose of a compound disclosed herein administered twice daily is about 1 to 50 mg/kg, 1 to 40 mg/kg, 2.5 to 40 mg/kg, 3 to 40 mg/kg, 3 to 35 mg/kg, most preferably 3 to 30 mg/kg. In embodiments of the invention, the required daily dose of the compound is about 1 to 80 mg/kg and within that range 1 to 70 mg/kg, 1 to 65 mg/kg, 2 to 70 mg/kg, 3 to 70 mg/kg, 4 to 65 mg/kg, 5 to 65 mg/kg, or 6 to 60 mg/kg.

In embodiments of the invention, the required dose of a compound disclosed herein, administered twice daily is about 1 to 50 mg/kg, 1 to 40 mg/kg, 2.5 to 40 mg/kg, 3 to 40 mg/kg, 3 to 35 mg/kg, most preferably 3 to 30 mg/kg.

In other embodiments of the invention, the required daily dose of a compound disclosed herein, is about 1 to 80 mg/kg and within that range 1 to 70 mg/kg, 1 to 65 mg/kg, 2 to 70 mg/kg, 3 to 70 mg/kg, 4 to 65 mg/kg, 5 to 65 mg/kg, or 6 to 60 mg/kg.

A composition for use in treatment of the invention may comprise a unit dosage of at least one compound of the invention to provide beneficial effects. A "unit dosage" or "dosage unit" refers to a unitary i.e. a single dose which is capable of being administered to a patient, and which may be readily handled and packed, remaining as a physically and chemically stable unit dose comprising either the active agents as such or a mixture with one or more solid or liquid pharmaceutical excipients, carriers, or vehicles.

A subject may be treated with a compound of the formula II or composition or formulation thereof on substantially any desired schedule. A composition for use in the invention may be administered one or more times per day, in particular 1 or 2 times per day, once per week, once a month or continuously. However, a subject may be treated less frequently, such as every other day or once a week, or more frequently. A compound, composition or formulation of the invention may be administered to a subject for about or at least about 1 week, 2 weeks to 4 weeks, 2 weeks to 6 weeks, 2 weeks to 8 weeks, 2 weeks to 10 weeks, 2 weeks to 12 weeks, 2 weeks to 14 weeks, 2 weeks to 16 weeks, 2 weeks to 6 months, 2 weeks to 12 months, 2 weeks to 18 months, or 2 weeks to 24 months, periodically or continuously.

A regimen for supplementing a human's diet may be provided, comprising administering to the human a supplement comprising a compound of the formula II, or nutraceutically acceptable derivatives thereof. A subject may be treated with a supplement at least about every day, or less frequently, such as every other day or once a week. A supplement of the invention may be taken daily but consumption at lower frequency, such as several times per week or even isolated doses, may be beneficial.

In a particular aspect, the regimen for supplementing a human's diet, may comprise administering to the human about 25 to about 200 milligrams of a compound disclosed herein, or nutraceutically acceptable derivatives thereof on a daily basis. In another aspect, about 50 milligrams of a compound of the formula II is administered to the human on a daily basis.

A supplement may be ingested with or after a meal. Thus, a supplement may be taken at the time of a person's morning meal, and/or at the time of a person's noontime meal. A portion may be administered shortly before, during, or shortly after the meal. For daily consumption, a portion of the supplement may be consumed shortly before, during, or shortly after the human's morning meal, and a second portion of the supplement may be consumed shortly before, during, or shortly after the human's noontime meal. The morning portion and the noontime portion can each provide

approximately the same quantity of a compound of the formula II. A supplement and regimens described herein may be most effective when combined with a balanced diet according to generally accepted nutritional guidelines, and a program of modest to moderate exercise several times a week.

The invention will be described in greater detail by way of specific examples. The following examples are offered for illustrative purposes.

EXAMPLES

**Example 1**

The following methods described in WO 2004/075882 (PCT/CA2004/000272) can be used to study the compounds of the invention:

**Mice.** Experimental groups of TgCRND8 mice [Chishti, M. A. et al., J.Biol Chem 276, 21562-21570 (2001); Janus,C. et al., Nature 408, 979-982 (2000)] will be initially treated with 5mg/Kg/day-300mg/Kg/day of a compound disclosed herein.

Two cohorts of animals (n=10 mice per treatment arm) will be entered into the study at 6 weeks to five months of age, and outcomes will be analyzed at 4 to 6 months of age. The body weight, coat characteristics and in cage behaviour will be monitored.

**Behavioural tests:** Morris Water Maze testing will be performed as described in Janus,C. *et al.,* 2000. After non-spatial pre-training, mice will undergo discrimination training for 5 days with 4-trials per day. Behavioral data will be analyzed using a mixed model of factorial analysis of variance (ANOVA) with drug or genotype and training sessions as repeated measure factors.

**Cerebral amyloid burden.** Brains will be removed and one hemisphere fixed in 4% paraformaldehyde and embedded in paraffin wax in the mid sagittal plane. To generate sets of systematic uniform random sections, 5 μm serial sections will be collected across the entire hemisphere. Sets of sections at 50 mm intervals will be used for analyses (10-14 sections/set). Plaques will be identified after antigen retrieval with formic acid, and incubated with primary anti-Aβ antibody (Dako M-0872), followed by secondary antibody (Dako StreptABCcomplex/horseradish kit). End products will be visualized with DAB and counter-stained with hematoxylin. Amyloid plaque burden will be assessed with Leco IA-3001 image analysis software interfaced with Leica microscope and Hitachi KP-M1U CCD video camera. Vascular amyloid burden will be similarly analyzed and a dissector will be used to measure the diameter of affected vessels.

**Plasma and Cerebral Aβ Content.** Hemi-brain samples will be homogenized in a buffered sucrose solution, followed by either 0.4% diethylamine/100mM NaCl for soluble Aβ levels or cold formic acid for the isolation of total Aβ. After neutralization, the samples will be diluted and analyzed for Aβ40 and Aβ42 using commercially available kits (BIOSOURCE International). Each hemisphere will be analyzed in triplicate and the mean values ± SEM reported. Western blot analyses will be performed on all fractions using urea gels for Aβ species analyses (Wiltfang, J. et al., J Neurochem 81, 481-496 (2002)). Aβ will be detected using 6E10 (BIOSOURCE International) and Enhanced Chemiluminenscence (Amersham).

**Gliosis Quantitation.** Five randomly selected, evenly spaced, sagittal sections will be collected from paraformaldehyde-fixed and frozen hemispheres of treated and control mice. Sections will be immunolabelled for astrocytes with anti-rat GFAP $IgG_{2a}$ (Dako; diluted 1:50) and for microglia with anti-rat CD68 $IgG_{2b}$ (Dako; 1:50). Digital images will be captured using a Coolsnap digital camera (Photometrics, Tuscon, Arizona) mounted to a Zeiss, Axioscope 2 Plus microscope. Images will be analysed using Openlab 3.08 imaging software (Improvision, Lexington MA).

**Survival Census:** The probability of survival will be assessed by the Kaplan-Meier technique (Haccou, P., & Mellis, E., Statistical Analysis of Behavioural Data, pg 120-186, Oxford University Press, Oxford (1995)), computing the probability of survival at every occurrence of death, making it suitable for small sample sizes. The Tarone-Ware test will be used to compare the treatments.

**Analysis of APP in brain.** Mouse hemi-brain samples will be homogenized and spun at 109,000 x g, in 20mM Tris pH 7.4, 0.25M sucrose, 1mM EDTA and 1mM EGTA, and a protease inhibitor cocktail, mixed with 0.4%DEA (diethylamine)/100mM NaCl. The supernatants will be analysed for APPs levels by Western blotting using mAb 22C11, while the pellets will be analysed for APP holoprotein with mAb C1/6.1 as described in Janus, 2000; Chishti, M, 2001.

**Results**

To assess their effectiveness *in vivo,* compounds disclosed herein will be administered to a murine model of Alzheimer's disease (TgCRND8) (Chishti, M. A. et al., J. Biol Chem 276, 21562-21570 (2001); Janus,C. et al., Nature 408, 979-982 (2000)). The TgCRND8 mice and non-transgenic littermates will be assigned to sex- and age-matched

cohorts that are then used to test the effectiveness of the compounds disclosed herein as therapeutics. The mice will be randomly assigned to receive active compound, mock therapy, or no therapy. The endpoints will be cognitive function, brain Aβ levels, and neuropathology.

The data are expected to show that compounds disclosed herein can prevent and reverse the AD-like phenotype in TgCRND8 mice, reducing cognitive deficits, amyloid plaques, amyloid angiopathy, Aβ-induced inflammatory response, and/or accelerated mortality. The levels of soluble Aβ oligomers are expected to be significantly reduced in the brain of mice treated with compounds disclosed herein.

**Example 2**

The compounds disclosed herein can be tested in an Alternating Lever Cyclic Ratio rat model of Alzheimer's disease (O'Hare, E. et al, Behavior Pharmacology, 7:742-753, (1996); Richardson, RL, et al., Brain Research, 54: 1-10, (2002)). This model has been able to detect cognitive deficits due to direct injection of amyloid-β oligomers into rat brain. The compounds can be administered concurrent with Aβ oligomers known to adversely affect cognition and their ability to counteract the oligomer-induced cognitive decline can be assessed.

In the Alternativing Lever Cyclic Ratio (ALCR) test rats must first learn a complex sequence of lever-pressing requirements in order to earn food reinforcement in a two-lever experimental chamber. Subjects must alternate between two levers by switching to the other lever after pressing the first lever enough to get food rewards. The exact number of presses required for each food reward changes, first increasing from 2 responses per food pellet up to 56 based on the quadratic function, $x^2$-x. One cycle is an entire ascending and descending sequence of these lever press requirements (e.g., 2, 6, 12, 20, 30, 42, 56, 56, 42, 30, 20, 12, 6, and 2 presses per food reward). Six such full cycles are presented during each daily session. Errors can be scored when the subject perseveres on a lever after pressing enough to get the food reward, i.e., does not alternate (a Perseveration Error), or when a subject switches levers before completing the response requirement on that lever (a Switching Error).

**Example 3**

Amyloid beta (Aβ) fibrils were prepared by the methods disclosed in Kheterpal, I et al, Biochemistry, 2001 40(39):11757 and Cannon MJ et al, Anal Biochem. 2004 328(1):67. The fibrils were immobilized on an affinity column and assayed by FAC-MS using the methods described in Leticia Toledo-Sherman, et al, J. Med. Chem. 2005, 48: 3221 or Slon-Usakiewicz J.J. et al, Clin. Proteom. J. 2004, 1:227-234. In particular, Aβ fibrils were immobilized to CBX1000C (COOH-modified) beads (Millipore) as follows. CBX1000C (5 mg) activated by reaction with EDAC/NHS in 0.1M MES buffer containing 0.5 M NaCl, pH 6.4. After 45 min of mixing at room temperature the beads were centrifuged and supernatant was removed and washed with 1X MES. The beads were resuspended in 250 μL of MES buffer and 100 μg of Aβ fibrils (in 1X PBS) was added. The mixture was incubated for 2h at room temperature and then overnight at 4°C with 360° vertical rotation followed by 1X PBS. After loading immobilized Aβ fibrils, the FAC-MS capillary columns (250 μm id x 2.5 cm) were washed with 50 μL (at 200 μL/h) of 1X PBS buffer followed by 50 μL of the running buffer (20 mM $NH_4OAc$ containing 1% DMSO). The activity of the immobilized amyloid fibrils was determined using Aβ monomer (1 μM) as the indicator and M3 (1 μM) as the void marker in 20 mM $NH_4OAc$ containing 1% DMSO. The makeup buffer was 90% methanol containing 0.1% acetic acid in water. Analyte solutions contained Aβ monomer (1 μM) as the indicator and M3 (1 μM) as the void marker and compounds (see Table 1) ranging from 1- 10 μM in 20 mM $NH_4OAc$ containing 1% DMSO. The flow rates used were 80 μL/h for the makeup buffer and 100 μL/h for the FAC-MS columns. The column was connected to an AB/Sciex API 3000 triple-quadrupole mass spectrometer (Concord, Ontario, Canada) and syringe pumps (Harvard Biosciences, Holliston, MA) and was allowed to equilibrate with the running buffer until the Aβ monomer (M+H) signal was stable, then data acquired. After 1 min, the system was switched to the analyte solution and data collection continued until the Aβ monomer signal had maximized for at least 10 min. The column was washed with running buffer until the Aβ monomer signal had reduced to its background level to regenerate the column. The data was analyzed using a customized Excel macro to determine the breakthrough times of amyloid beta and M3.

The % shift is determined from the equation:

$$\% \text{ Shift} = (t_I - t)/(t_I - t_{NSB}) \times 100\%$$

where t is the breakthrough time difference, measured at the inflection point, of the sigmoidal fronts between the indicator and void marker in the presence of any competing ligand(s), $t_{NSB}$ is the non-specific breakthrough time difference in the absence of immobilized target (and is a constant for the indicator used) and $t_I$ is the breakthrough time difference in the absence of any competing ligands.

The FAC-MS % shift results of the free Aβ monomer assayed with immobilied Aβ fibrils in the presence of various compounds at 1 and 10 μM is shown in Tables 1 and 2.

**Example 4** (for reference only)

Mono-substituted scyllo-inositols (methyl, ethyl, benzyl, and trifluoromethyl) were synthesized as follows. A mono-methyl scyllo-inositol **(9)** was synthesized starting from myo-inositol **(1)** as described in the literature and illustrated in Figure 1. The literature protocol for the methylation of the intermediate **6** on a 600 mg scale afforded ~230 mg of the pure **7** and ~300 mg of the recovered un-reacted starting material. The structure of **7** was confirmed by $^1$H-NMR. ~45 mg of methyl-scyllo-inositol was synthesized and identified by $^1$H-NMR and MS analysis.

Alkylation of the intermediate **6** with EtI and BnBr was done on a 600 mg scale starting with **6.** The products were purified by column chromatography and identified by $^1$H-NMR. The intermediate **8** (Me and Bn) and the ethyl analog of **8** were also synthesized. ~120 mg of benzyl-scyllo-inositol was synthesized and identified by $^1$H-NMR and MS analysis.

Trifluoromethyl-scyllo-inositol was synthesized from intermediate **6** similar to the mono-methyl-scyllo- inositol (see Figure 3). The fact, that trifluoroiodomethane is a gas required some modifications to the original protocol. Thus the solution of intermediate **6** in DMF was saturated with $CF_3I$ at low temperature, then sodium hydride was added and the reaction vessel sealed. A vigorous evolution of a gas was observed, but no changes in the reaction progress were observed at low temperature.

Di-substituted scyllo-inositols (1,3-dimethyl and 1,3-diacetyl) were synthesized using a process similar to the process for producing methyl-scyllo-inositol starting from intermediate **6.** A five-step reaction scheme for the synthesis of di-substituted scyllo-inositols from intermediate **6** is illustrated in Figure 2.

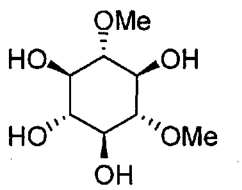

1,3-dimethyl-scyllo-inositol

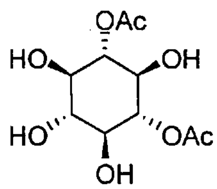

1,3-diacetyl-scyllo-inositol

# Figure 1

## Scheme 1

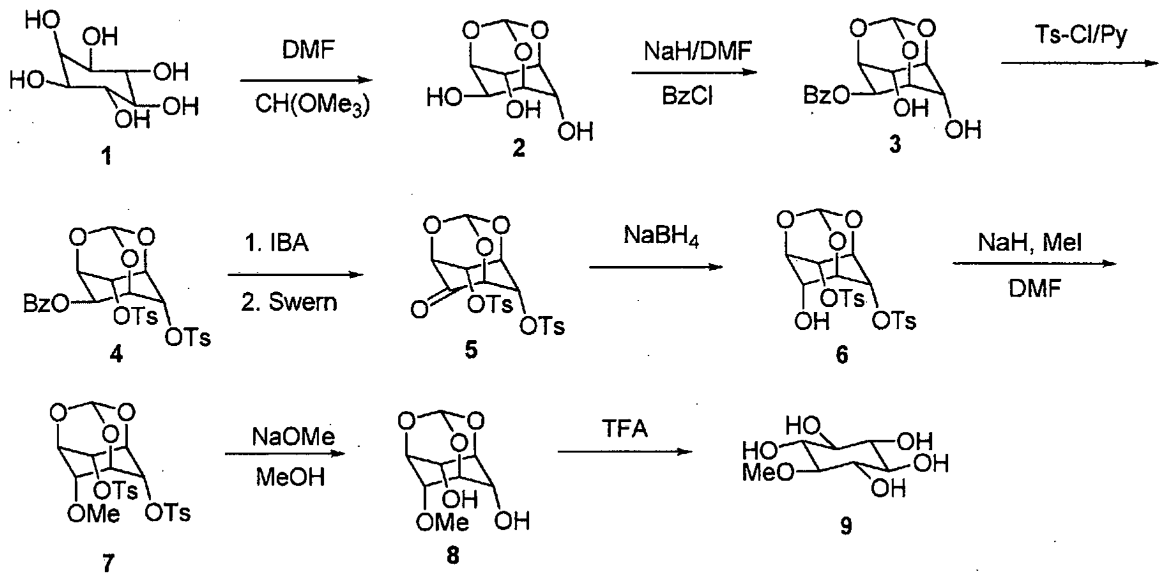


1 2 3 4 5 6 7 8 9

Abbreviations: $CH(OCH_3)_3$ = trimethyl orthoformate, DMF = dimethyl formamide; NaH=sodium hydride, BzCl = benzyl chloride; IBA = iodosobenzoic acid, Swen is a Swern oxidation; $NaBH_4$ = sodium borohydride; MeI=methyl iodide; NaOMe = sodium methoxide, MeOH = methanol; TFA is trifluoroacetic acid.

## Figure 2

**Scheme 2**

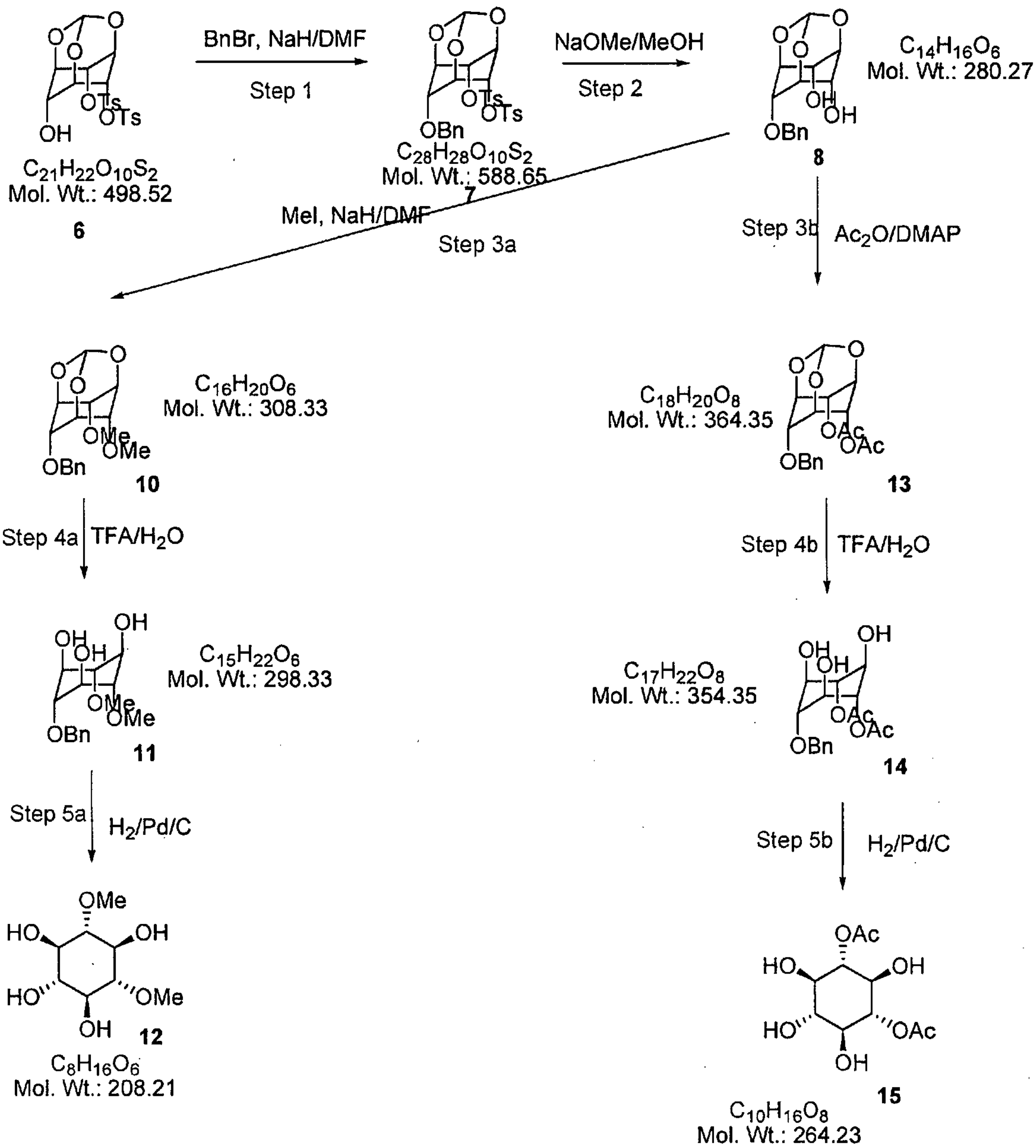


Abbreviations: Structure 6 –OTs= p-toluenesulfonate; BnBr = benzyl bromide; Step 3b –DMAP = 4-Dimethylaminopyridine, $Ac_2O$ = acetic anhydride; Steps 5a, 5b – $H_2$/Pd/C = hydrogen and palladium on carbon; all other abbreviations as defined in Figure 1.

## Figure 3

**Scheme 3**

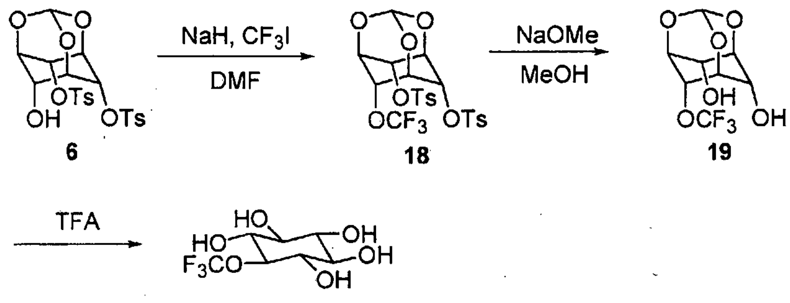


**6** **18** **19**

**20**

Abbreviation: $CF_3I$ = trifluoromethyl iodide. All other abbreviations as in Figures 1 and 2.

**TABLE I**

| Structure | Structure Composition | Molec. Formula | Mol Weight Structure | Name | Source | ABeta Shift |
|---|---|---|---|---|---|---|
| 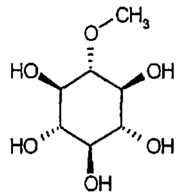 | C 43.30%<br>H 7.27%<br>O 49.44% | | 194.18603 | Methyl-scyllo-inositol | Dalton | 48 |
| 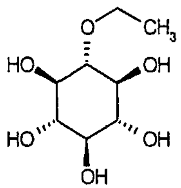 | C 46.15%<br>H 7.75%<br>O 46.10% | $C_8H_{16}O_6$ | 208.21312 | Ethyl-scyllo-inositol | Dalton | 14 |
| 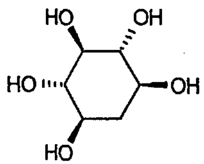 | C 43.90%<br>H 7.37%<br>O 48.73% | $C_6H_{12}O_5$ | 164.15954 | 1,3,5/2,4-pentahydroxy cyclohexane (Scyllo-Quercitol) | | 15 |
| 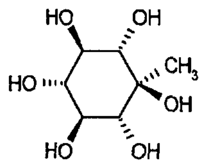 | C 43.30%<br>H 7.27%<br>O 49.44% | $C_7H_{14}O_6$ | 194.18603 | 1-<Methyl-1,3,5/2,4,6-Inositol (Mytilitol) | | 22 |
| 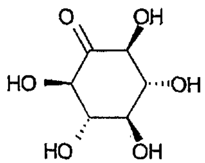 | C 40.45%<br>H 5.66%<br>O 53.89% | $C_6H_{10}O_6$ | 178.143 | 2,4,5/3,5-Pentahydroxy cyclohexanon e (Scyllo-inosose) | | 11 |
| 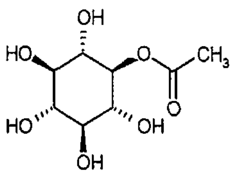 | C 43.25%<br>H 6.35%<br>O 50.40% | $C_8H_{14}O_7$ | 222.19658 | 1-acetyl-scyllo-inositol | Dalton | 31 |

(continued)

| Structure | Structure Composition | Molec. Formula | Mol Weight Structure | Name | Source | ABeta Shift |
|---|---|---|---|---|---|---|
| 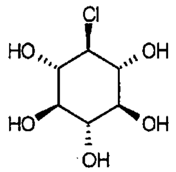 | C 36.29%<br>H 5.58%<br>Cl 17.85%<br>O 40.28% | $C_6H_{11}Cl\ O_5$ | 198.60457 | 1-Chloro-1-deoxy-scyllo-inositol | V-Labs | 67 |

**TABLE 2**

| Structure | Structure Composition | SOURCE | ABeta SHIFT |
|---|---|---|---|
| 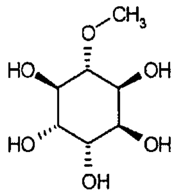 | C 43.30% H 7.27% O 49.44% | SIGMA-ALDRICH | 3 |
| 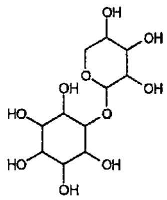 | C 42.31 % H 6.46% O 51.23% | SIGMA-ALDRICH | 3 |
| 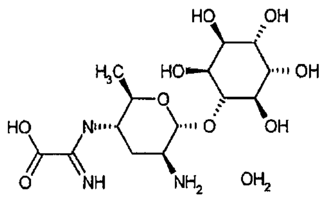 | C 42.32% H 6.85% N 10.57% O 40.26% | SIGMA-ALDRICH | 2 |
| 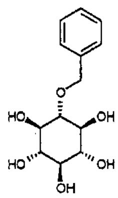 | C 57.77% H 6.71% O 35.52% | DALTON | 3 |
| 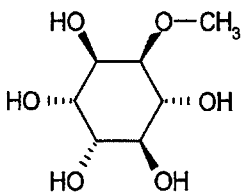 | C 43.30% H 7.27% O 49.44% | | 5 |
| 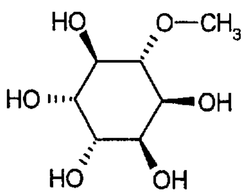 | C 43.30% H 7.27% O 49.44% | | 2 |
| 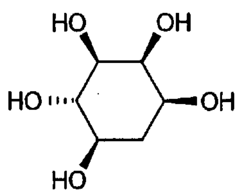 | C 43.90% H 7.37% O 48.73% | | 4 |
| 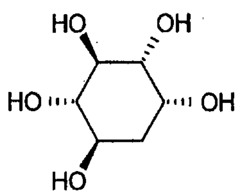 | C 43.90% H 7.37% O 48.73% | | 5 |

(continued)

| Structure | Structure Composition | SOURCE | ABeta SHIFT |
|---|---|---|---|
| 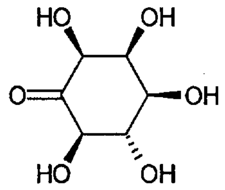 | C 40.45% H 5.66% O 53.89% | | 7 |
| 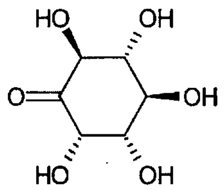 | C 40.45% H 5.66% O 53.89% | | 5 |
| 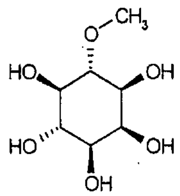 | C 43.30% H 7.27% O 49.44% | IRL | 4 |
| 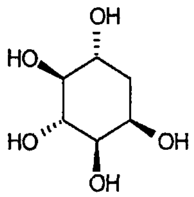 | C 43.90% H 7.37% O 48.73% | IRL | 2 |
| 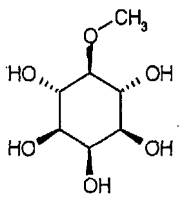 | C 43.30% H 7.27% O 49.44% | IRL | 3 |

## Claims

1. A pharmaceutical composition for use in the treatment or prevention of Alzheimer's disease comprising a therapeutically effective amount of a compound of the formula II

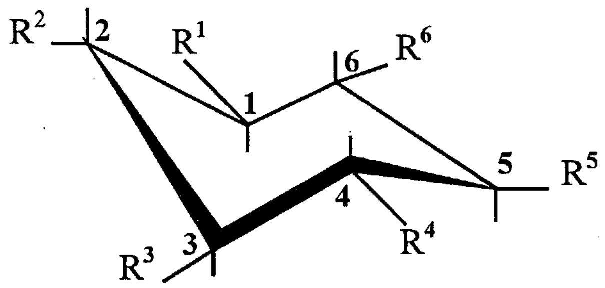

II

wherein five of $R^1$, $R^2$, $R^3$, $R^4$, $R^5$, and $R^6$ are hydroxyl and one of $R^1$, $R^2$, $R^3$, $R^4$, $R^5$, and $R^6$ is fluoro.

2. A pharmaceutical composition for the use according to claim 1 wherein the Alzheimer's disease is a presenile form.

3. A pharmaceutical composition for the use according to claim 1 wherein the Alzheimer's disease is a senile form.

4. A pharmaceutical composition according to any preceding claim for use in the treatment or prevention of mild cognitive impairment.

5. Use of a pharmaceutical composition or a therapeutically effective amount of a compound of the formula II as defined in claim 1 in the manufacture of a medicament for the treatment or prevention of Alzheimer's disease in a subject.

**Patentansprüche**

1. Pharmazeutische Zusammensetzung zur Verwendung in der Behandlung oder Vorbeugung von Morbus Alzheimer, umfassend eine therapeutisch wirksame Menge einer Verbindung der Formel II

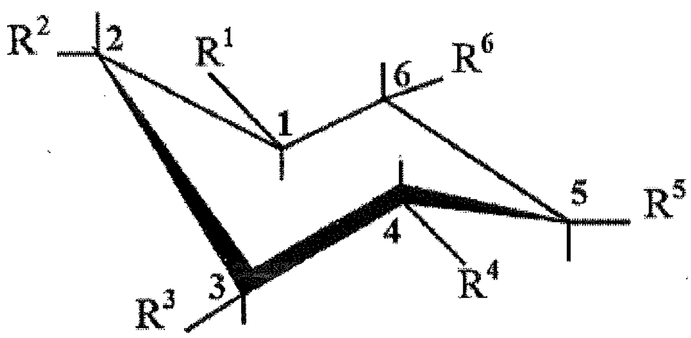

II

worin fünf von $R^1$, $R^2$, $R^3$, $R^4$, $R^5$ und $R^6$ Hydroxyl bedeuten und eines von $R^1$, $R^2$, $R^3$, $R^4$, $R^5$ und $R^6$ Fluor bedeutet.

2. Pharmazeutische Zusammensetzung zur Verwendung nach Anspruch 1, wobei der Morbus Alzheimer eine präsenile Form aufweist.

3. Pharmazeutische Zusammensetzung zur Verwendung nach Anspruch 1, wobei der Morbus Alzheimer eine senile Form aufweist.

4. Pharmazeutische Zusammensetzung nach einem vorhergehenden Anspruch zur Verwendung in der Behandlung oder Vorbeugung von milder Beeinträchtigung der kognitiven Fähigkeiten.

5. Verwendung einer pharmazeutischen Zusammensetzung oder einer therapeutisch wirksamen Menge einer Verbindung der Formel II wie in Anspruch 1 definiert in der Herstellung eines Arzneimittels für die Behandlung oder Vorbeugung von Morbus Alzheimer in einem Individuum.

## Revendications

1. Composition pharmaceutique destinée à une utilisation dans le traitement ou la prévention de la maladie d'Alzheimer, comprenant une quantité thérapeutiquement efficace d'un composé de formule II

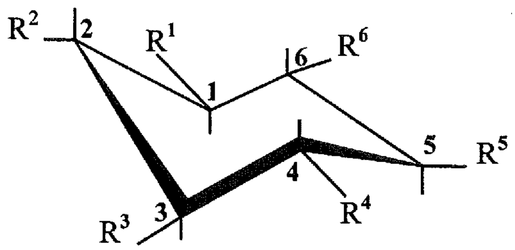

II

dans laquelle cinq parmi $R^1$, $R^2$, $R^3$, $R^4$, $R^5$ et $R^6$ sont hydroxyle et l'un parmi $R^1$, $R^2$, $R^3$, $R^4$, $R^5$ et $R^6$ est fluoro.

2. Composition pharmaceutique pour l'utilisation selon la revendication 1, dans laquelle la maladie d'Alzheimer est une forme présénile.

3. Composition pharmaceutique pour l'utilisation selon la revendication 1, dans laquelle la maladie d'Alzheimer est une forme sénile.

4. Composition pharmaceutique selon l'une quelconque des revendications précédentes, destinée à une utilisation dans le traitement ou la prévention d'un trouble cognitif léger.

5. Utilisation d'une composition pharmaceutique ou d'une quantité thérapeutiquement efficace d'un composé de formule II tel que défini selon la revendication 1, dans l'élaboration d'un médicament destiné au traitement ou à la prévention de la maladie d'Alzheimer chez un sujet.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004075882 A
- CA 2004000272 W


### Non-patent literature cited in the description


- **BOUVEAULT L.** *Bull. La Societe Chimique Paris,* vol. 11, 44-147
- **MICHAELIS T et al.** *NMR in Biomedicien,* 1993, vol. 6, 105-109
- **S. M. BERGE et al.** *J. Pharmaceutical Sciences,* 1977, vol. 66, 1
- **GREENE ; WUTS.** Protective Groups In Organic Synthesis. Wiley and Sons, 1991
- **FIESER ; FIESER'S.** Reagents for Organic Synthesis. John Wiley and Sons, 1991, vol. 1-17
- Rodd's Chemistry of Carbon Compounds. Elsevier Science Publishers, 1989, vol. 1-5
- Organic Reactions. John Wiley and Sons, 1991, vol. 1-40
- **MARCH J.** Advanced Organic Chemistry. John Wiley and Sons
- **LAROCK.** Comprehensive Organic Transformations. VCH Publishers, 1989
- **HUSSON, C. et al.** *Carbohyrate Research,* 1998, vol. 307, 163-165
- **SARMAH, M.P. ; SHASHIDAR, M.S.** *Carbohydrate Research,* 2003, vol. 338, 999-1001
- **CHISHTI, M. A. et al.** *J.Biol Chem,* 2001, vol. 276, 21562-21570
- **JANUS,C. et al.** *Nature,* 2000, vol. 408, 979-982
- **WILTFANG, J. et al.** *J Neurochem,* 2002, vol. 81, 481-496
- **HACCOU, P. ; MELLIS, E.** Statistical Analysis of Behavioural Data. Oxford University Press, 1995, 120-186
- **CHISHTI, M. A. et al.** *J. Biol Chem,* 2001, vol. 276, 21562-21570
- **O'HARE, E. et al.** *Behavior Pharmacology,* 1996, vol. 7, 742-753
- **RICHARDSON, RL et al.** *Brain Research,* 2002, vol. 54, 1-10
- **KHETERPAL, I et al.** *Biochemistry,* 2001, vol. 40 (39), 11757
- **CANNON MJ et al.** *Anal Biochem.,* 2004, vol. 328 (1), 67
- **LETICIA TOLEDO-SHERMAN et al.** *J. Med. Chem.,* 2005, vol. 48, 3221
- **SLON-USAKIEWICZ J.J. et al.** *Clin. Proteom. J.,* 2004, vol. 1, 227-234